# EUROPEAN PATENT SPECIFICATION

(11) **EP 1 929 961 B1**
(45) Date of publication and mention of the grant of the patent: **23.02.2011**
(21) Application number: 07254703.7
(22) Date of filing: 05.12.2007
(51) Int. Cl.: A61B 17/06, A61B 17/00

(54) **Knotless wound closure device**
Knotenlose Wundverschlussvorrichtung
Dispositif de fermeture de plaies sans noeuds

(30) Priority: 05.12.2006 US 567129
(43) Date of publication of application: 11.06.2008
(60) Divisional application: 10166547.9
(73) Proprietor: Tyco Healthcare Group, LP, North Haven, CT 06473 (US)
(72) Inventor: Maiorino, Nicholas, Branford CT 06405 (US); Primavera, Michael, Orange CT 06477 (US); Buchter, Mark S., Ansonia CT 06401 (US); Cohen, Matthew D., Berlin CT 06037 (US)
(74) Representative: Alcock, David

(56) References cited:
- EP-A- 0 632 999
- EP-A- 1 669 093
- WO-A-01/52751
- US-A- 5 123 913
- US-A- 5 814 056

## Description

### TECHNICAL FIELD

The present disclosure generally relates to wound closure devices having an uninterrupted or continuous stitch.

### Background of Related Art

For many years, surgeons have sealed tissue wounds using various wound closure devices and methods. Suturing is a surgical technique involving the connection of tissue by stitching the tissue together with a strand of appropriate suturing material.

Typically, a suture is prepared by piercing a needle with the suture attached through tissue on both sides of a wound, by pulling the ends of the suture to bring the sides of the wound together, and tying the suture into a knot. The knot preserves the tension on the suture to maintain the sides of the wound in approximation and allow the tissue to heal. An improperly tied knot can slip and untie at a tension far lower than the tension required to break the suture. When the suture is internal to the body, replacement of the failed suture can require another surgery.

A variety of devices have been developed for the transcutaneous placement, tying, and tightening of suture knots through a tissue tract. Despite the skill and due care involved in placing, tying, and tightening a suture knot using these devices, seepage of blood and fluids at the suture site and into the tissue tract can still occur.

US 5,123,913 discloses a suture device having a thread member comprising projections, a loop member, the loop member defining an opening having a first opening and a second opening connected by an access channel. US 5,123,913 discloses a wound closure device which can lock by passing the proximal end of the suture through the larger open portion of the loop member. The elongated body of the suture (which does not contain any openings) is then translated along the channel into the smaller portion of the opening of the loop member. There is thus a single area within which a lock can be formed with the projections, the area being provided by the size of the opening reducing as a result of the channel formed by protuberances.

Thus, there remains a need for fast and straightforward systems and methods to achieve wound closure, which are substantially free of blood or fluid leakage about the wound closure site. There also remains a need for a wound closure device utilized as a continuous stitch which can have multi-use per patient instead of multiple sutures used for one wound closure. Furthermore, there still remains a need for a self anchoring wound closure device having a secure locking mechanism.

### SUMMARY

The present invention relates to claim 1. A plurality of through-holes are disposed along the length of the elongated flexible body wherein the proximal end is configured and dimensioned to pass through body tissue and thereafter be selectively passed through at least one of the plurality of through-holes such that at least one of the surface features also passes through at least one through-hole and thereby forming a locked closed loop to secure body tissue held therein.

The present disclosure also provides an exemplary method of closing a wound which includes using such wound closure devices by passing the proximal end of the device through body tissue at least once and subsequently passing the proximal end through at least one of the plurality of through-holes and forming a locked closed loop to secure body tissue held therein.

### BRIEF DESCRIPTION OF THE DRAWINGS

Various embodiments of the present disclosure will be described hereinbelow with reference to the figures wherein:
FIG. 1 is a perspective view of a knotless wound closure device attached to a needle in accordance with the present disclosure;
FIGS. 2A-D are top plan views of alternative embodiments of the plurality of through holes of FIG. 1 in accordance with the present disclosure;
FIGS. 3A-I are cross sectional views of alternative embodiments of the elongated flexible body and surface features of the embodiment of FIG. 1 in accordance with the present disclosure; and
FIGS. 4-7 illustrate a series of steps employing the method of closing a wound in accordance with the present disclosure.

### DETAILED DESCRIPTION

Described herein are knotless wound closure devices utilized to form a continuous stitch and having an opposing anchoring point to function properly. The knotless wound closure device of the present disclosure eliminates the failure rate caused at or near the anchor point which would have caused support of the tissue approximation to be lost and seepage of blood and fluids to occur. The unique geometry and use of the knotless wound closure device also reduces or eliminates patient discomfort caused by the sharp protrusions which may be felt with a barbed suture device.

Accordingly, the knotless wound closure device is created with a specific geometry, namely with a plurality of surface features which interface with uniquely matching through-holes created through the length of the longitudinal axis of the elongated flexible body of the wound closure device which function as a unidirectional locking mechanism.

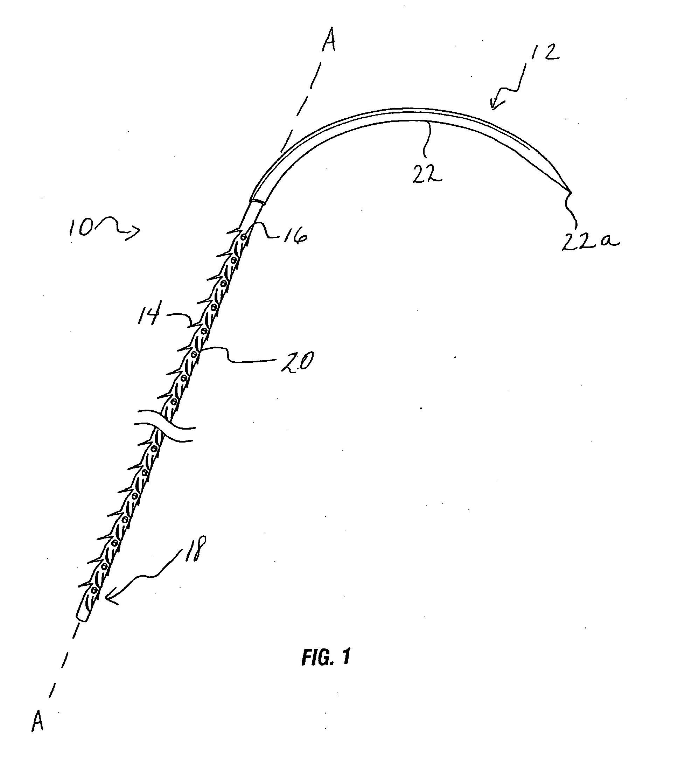

Referring now in detail to the drawings in which like reference numerals are applied to like elements in the various views, knotless wound closure device 10 is shown in FIG. 1 and generally includes an elongated flexible body 20 having a proximal end 12 with a needle 22 attached flexible body 20 and a distal end 18 and defining a longitudinal axis A-A. A plurality of surface features 14 extend generally away from the longitudinal axis A-A. Additionally, plurality of through-holes 16 are formed along the length of the elongated flexible body 20 wherein the needle 22 includes a sharpened tip 22a which is configured and dimensioned to pass through body tissue and thereafter be selectively passed through at least one of the plurality of through-holes 16 such that at least one of the surface features 14 also passes through the at least one through-hole 16 thereby forming a locked closed loop to secure body tissue held therein.

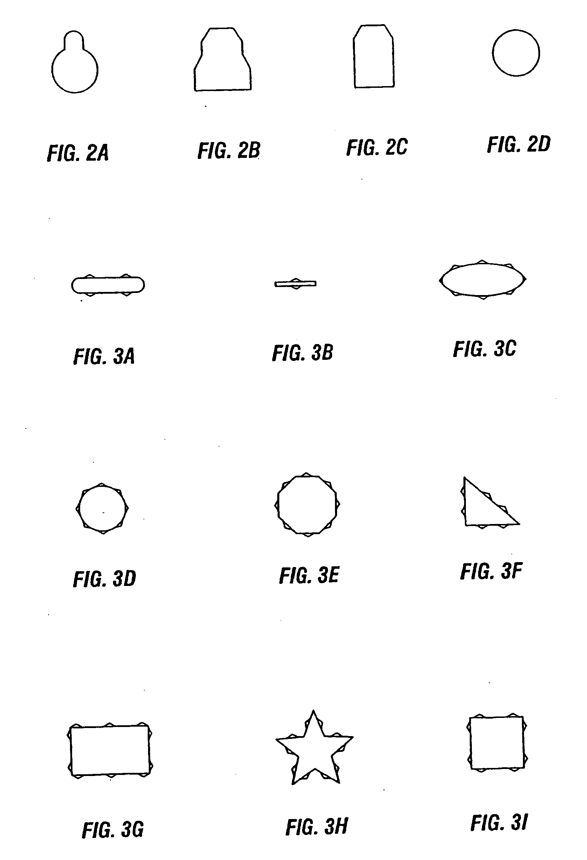

Referring to FIGS. 2A-D, in various alternative embodiments, the cross-sectional geometry of the plurality of through-holes 16 may consist of a key-shape (FIG. 2A), a compound wedge (FIG. 2B), a wedge (FIG. 2C) and a circle (FIG. 2D).

Referring now to FIGS. 3A-I, in various alternative embodiments, the cross-sectional geometry of the elongated flexible body 20 may consist of an oval shape (FIG. 3A), a flat tape shape (FIG. 3B), an elliptical shape (FIG. 3C), a round shape (FIG. 3D), an octagon shape (FIG. 3E), an oblique shape (FIG. 3F), a rectangle shape (FIG. 3G), a star shape (FIG. 3H) and a square (FIG. 3I).

In alternative embodiments, the surface features 14 may consist of barbs, hooks, latches, protrusions, leaves, teeth and/or combinations thereof.

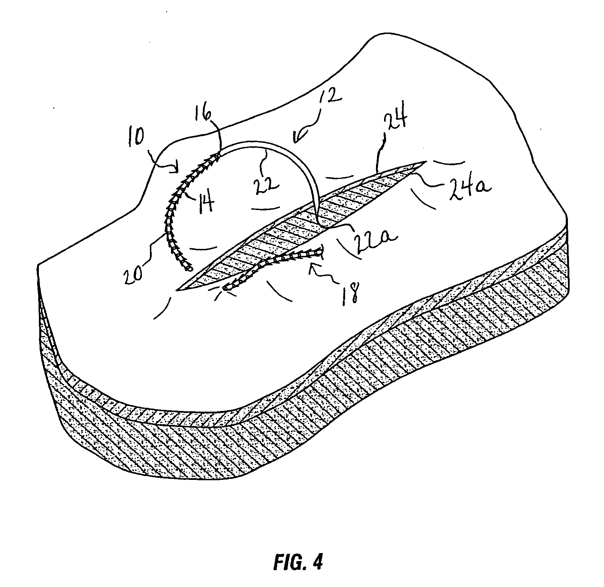

Referring to FIGS. 4-7, a series of steps or processes for an illustrative method of closing a wound using the knotless wound closure device described above is shown. In use, surface features 14 are interlaced through plurality of through-holes 16 penetrating through the longitudinal axis of elongated flexible body 20 and securing through a friction fit. Thus, as tension is applied to the device, a wedging action occurs thereby resulting in a more secure locking mechanism. The knotless wound closure device 10 and method of closing the wound is intended for general wound closure and can be utilized as either an "uninterrupted" or "continuous" stitch. The device will also support multi single unit use per patient.

In FIG.4, proximal end 12 of knotless wound closure device 10 is shown having penetrated both wound edges 24, 24a and approaching at least one of the plurality of through holes 16 of knotless wound closure device 10.

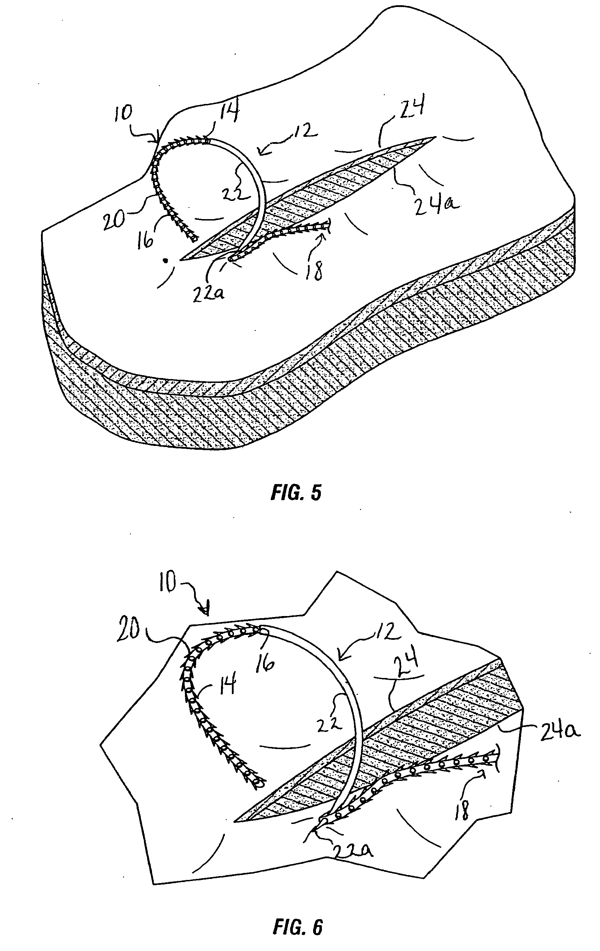

In FIG. 5, proximal end 12 is shown about to penetrate through one of the plurality of through holes 16.

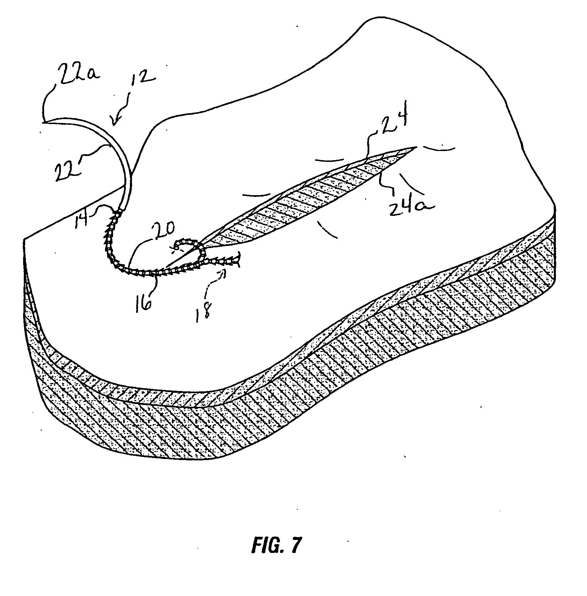

In FIG. 6, the proximal end 12 is shown penetrating through a through-hole 16. As proximal end 12 penetrates fully through the through-hole 16 (not shown) and the suture is pulled through through-hole 16, surface features 14 are compressed as they pass through through-hole 16 of knotless wound closure device 10 and expand upon exiting the other side of through-hole 16 thereby preventing reversal of the suture back through the through-hole 16. Additionally, the cross sectional dimension of flexible body 20 and the diameter of through-hole 16 may be formed so as to create an interference or friction fit between the two thereby securing device 10 through a friction fit as shown in FIG. 7.

In various embodiments, the knotless wound closure device may be constructed from materials selected from the group consisting of surgical fibers, sutures, filaments, tapes, slit sheets, and ribbons.

In embodiments, a suture in accordance with the present disclosure may be of monofilament or multifilament construction. The suture may have both a proximal and distal end, with barbs projecting from the elongated body towards at least one end thereby forming an included angle of less than about 90 degrees between the barbs and the suture body. Additionally, a bioactive agent may be deposited within the barb angles, that is, the angle formed between the barb and suture surface. Placement of a bioactive agent in the angle formed between the barbs and suture surface places the bioactive agent at precisely defined locations within a tissue wound closure, which thereby provides a unique controlled and sustained release dosage form.

The wound closure device in accordance with the present disclosure may be formed of degradable materials, non-degradable materials, and combinations thereof. Suitable degradable materials which may be utilized to form the device include natural collagenous materials or synthetic resins including those derived from alkylene carbonates such as trimethylene carbonate, tetramethylene carbonate, and the like, caprolactone, dioxanone, glycolic acid, lactic acid, glycolide, lactide, homopolymers thereof, copolymers thereof, and combinations thereof. In some embodiments, glycolide and lactide based polyesters, especially copolymers of glycolide and lactide, may be utilized to form a suture of the present disclosure.

Suitable non-degradable materials which may be utilized to form the device of the present disclosure include polyolefins, such as polyethylene, polypropylene, copolymers of polyethylene and polypropylene, and blends of polyethylene and polypropylene; polyamides (also known as nylon); polyesters such as polyethylene terephthalate; polytetrafluoroethylene; polyether-esters such as polybutester; polytetramethylene ether glycol; 1,4-butanediol; polyurethanes; and combinations thereof. In other embodiments, non-degradable materials may include silk, cotton, linen, carbon fibers, and the like. In some useful embodiments, polypropylene can be utilized to form the suture. The polypropylene can be isotactic polypropylene or a mixture of isotactic and syndiotactic or atactic polypropylene.

Filaments used for forming wound closure devices of the present disclosure may be formed using any technique within the purview of those skilled in the art, such as, for example, extrusion, molding and/or solvent casting. In embodiments, the strands can be extruded through an extruder unit of a conventional type, such as those disclosed in U.S. Pat. Nos. 6,063,105; 6,203,564; and 6,235,869..

The device of the present disclosure may include a yarn made of more than one filament, which may contain multiple filaments of the same or different materials. Where the device is made of multiple filaments, the suture can be made using any known technique such as, for example, braiding, weaving or knitting, each of which may be formed by any suitable method within the purview of those skilled in the art. The filaments may also be combined to produce a non-woven suture. The filaments themselves may be drawn, oriented, crinkled, twisted, commingled or air entangled to form yarns as part of the suture forming process.

Once the device is constructed, it can be sterilized by any means within the purview of those skilled in the art.

Wound closure devices in accordance with the present disclosure may be coated or impregnated with one or more medico-surgically useful substances, e.g., bioactive agents which accelerate or beneficially modify the healing process when the device is applied to a wound or surgical site. Suitable bioactive agents include, for example, biocidal agents, antibiotics, antimicrobial agents, medicants, growth factors, anti-clotting agents, analgesics, anesthetics, anti-inflammatory agents, wound repair agents and the like, and combinations thereof. Bioactive agents may be applied onto the wound closure device of the present disclosure utilizing any method within the purview of one skilled in the art including, for example, dipping, spraying, vapor deposition, brushing, compounding and the like.

Surface features may be formed on the elongated flexible body of the wound closure device utilizing any method within the purview of one skilled in the art. Such methods include, but are not limited to, cutting, molding, and the like. In some embodiments, surface features may be formed by making with acute angular cuts directly into the elongated flexible body, with cut portions pushed outwardly and separated from the body. The depth of the surface features thus formed generally away from the elongated flexible body may depend on the diameter of the material and the depth of the cut. In some embodiments, a suitable device for cutting a plurality of axially spaced surface features on the exterior of a filament may use a cutting bed, a cutting bed vise, a cutting template, and a blade assembly to perform the cutting. In operation, the cutting device has the ability to produce a plurality of axially spaced surface features such as barbs in the same or random configuration and at different angles in relation to each other. Other suitable methods of cutting the barbs include the use of a laser or manual methods. The device can be packaged in any number of desired pre-cut lengths and in pre-shaped curves.

In various embodiments, all of the surface features may be aligned to allow the elongated body of the knotless wound closure device to move through tissue in one direction and resist moving through tissue in the opposite direction. For example, referring to FIG. 1, the surface features 14 on elongated body 20 may be formed into a single directional wound closure device 10. In embodiments elongated body 20 may be attached to needle 22. The surface features 14 permit movement of device 10 through tissue in the direction of movement of a needle end 22 but are generally rigid in an opposite direction and prevent movement of device 10 in a direction opposite the direction of movement of a needle end 22.

In other embodiments, the surface features may be aligned on a first portion of a length of a body to allow movement of a first end of the device through tissue in one direction, while surface features on a second portion of the length of the body may be aligned to allow movement of the second end of the device in an opposite direction.

The surface features may be arranged in any suitable pattern, for example, in a helical pattern. The number, configuration, spacing and surface area of the surface features can vary depending upon the tissue in which the wound closure device is used, as well as the composition and geometry of the material utilized to form the wound closure device. Additionally, the proportions of the surface features may remain relatively constant while the overall length of the surface features and the spacing of the surface features may be determined by the tissue being connected. For example, if the wound closure device is to be used to connect the edges of a wound in skin or tendon, the surface features may be made relatively short and more rigid to facilitate entry into this rather firm tissue. Alternatively, if the wound closure device is intended for use in fatty tissue, which is relatively soft, the surface features may be made longer and spaced further apart to increase the ability of the wound closure device to grip the soft tissue.

The surface area of the surface features can also vary. For example, fuller-tipped surface features can be made of varying sizes designed for specific surgical applications. For joining fat and relatively soft tissues, larger surface features may be desired, whereas smaller surface features may be more suitable for collagen-dense tissues. In some embodiments, a combination of large and small surface features within the same structure may be beneficial, for example when a wound closure device is used in tissue repair with differing layer structures. Use of the combination of large and small surface features with the same wound closure device wherein barb sizes are customized for each tissue layer will ensure maximum anchoring properties. In certain embodiments, a single directional wound closure device as depicted in FIG. 1 may have both large and small surface features; in other embodiments a bi-directional wound closure device (not shown) may have both large and small surface features.

The wound closure device of the present disclosure may be utilized for all wound closure techniques and tissue connection procedures. Procedures can include endoscopic techniques, plastic and reconstructive surgeries, general wound closure, cardiovascular tissues, orthopedics, obstetrics, gynecology and urology. Typical tissue types include the various layers of muscle, ligaments, tendons, fascia, fat and/or skin.

There is also described a method of closing a wound comprising the steps of:
providing the knotless wound closure device as described herein and passing the proximal end of the knotless wound closure device through body tissue at least once and subsequently passing the proximal end through at least one of the plurality of through-holes thereby forming a locked closed loop to secure body tissue held therein.
The passing step may include passing the proximal end through body tissue a plurality of times before passing the proximal end through at least one of the plurality of through-holes thereby forming an uninterrupted stitch.

A knotless wound closure device includes an elongated flexible body with proximal and distal ends having a plurality of through-holes along its length and defining a longitudinal axis with a plurality of surface features extending away from the axis. The proximal end is configured and dimensioned to pass through body tissue and thereafter be selectively passed through at least one of the plurality of through-holes such that at least one of the surface features also passes through the through-hole thereby forming a locked closed loop to secure body tissue held therein.

While the above description contains many specifics, these specifics should not be construed as limitations on the scope of the disclosure, but merely as exemplifications of embodiments thereof. Those skilled in the art will envision many other possibilities within the scope of the disclosure as defined by the claims appended hereto.

## Claims

1. A knotless wound closure device (10) comprising:
an elongated flexible body (20) having a proximal end (12) and a distal end (18), the elongated flexible body (20) defining a longitudinal axis (A-A);
a plurality of surface features (14) extending generally away from the longitudinal axis;
a plurality of through-holes (16) formed along the length of the elongated flexible body (20);
wherein the proximal end (12) is configured and dimensioned to pass through body tissue and thereafter be selectively passed through at least one of the plurality of through-holes (16) such that at least one of the surface features (14) also passes through the at least one through-hole (16) thereby forming a locked closed loop to secure body tissue held therein
wherein the knotless wound closure device (10) has a specific geometry such that each of the plurality of surface features (14) is adapted to interface with any of said through holes, wherein said through-holes are uniquely matching through holes created through the length of the elongated flexible body (20), to function as a uni-directional locking mechanism.

2. The knotless wound closure device (10) of claim 1, wherein the device is constructed from materials selected from the group consisting of surgical fibers, sutures, filaments, tapes, slit sheets, and ribbons.

3. The knotless wound closure device (10) of claim 1, wherein the cross-sectional geometry of the plurality of through-holes (16) is selected from the group consisting of a key-shape, a wedge, a circle and a compound wedge.

4. The knotless wound closure device (10) of claim 1, wherein the cross-sectional geometry of the elongated flexible body (20) is selected from the group consisting of an oval, a rectangle, all ellipse, a circle, a square, a star, an oblique and an octagon.

5. The knotless wound closure device (10) of claim 1, wherein the surface features (14) are selected from the group consisting of barbs, hooks, latches, protrusions, leaves, teeth and/or combinations thereof.

6. The knotless wound closure device (10) of claim 1, wherein the proximal end (12) further comprises a needle (22) secured thereto.

7. The knotless wound closure device (10) of claim 1, wherein the surface features (14) includes a bioactive agent within an included angle of the surface feature (14) and the elongated flexible body (20).

8. The knotless wound closure device (10) of claim 7, wherein the bioactive agent is selected from the group consisting of biocidal agents, antibiotics, antimicrobial agents, medicaments, growth factors, anti-clotting agents, anesthetics, anti-inflammatory agents, wound repair agents and combinations thereof.

## Patentansprüche

1. Knotenlose Wundverschlussvorrichtung (10), welche aufweist:
einen länglichen flexiblen Körper (20) mit einem proximalen Ende (12) und einem distalen Ende (18), wobei der längliche flexible Körper (20) eine Längsachse (A-A) definiert;
eine Mehrzahl von Oberflächenmerkmalen (14), welche sich im Wesentlichen von der Längsachse wegerstrecken;
eine Mehrzahl von Durchgangslöchern (16), welche entlang der Länge des länglichen flexiblen Körpers (20) ausgebildet sind;
wobei das proximale Ende (12) derart ausgestaltet und dimensioniert ist um durch das Körpergewebe und anschließend auswählbar durch zumindest eines aus der Mehrzahl der Durchgangslöcher (16) hindurchgeführt zu werden, so dass zumindest eines der Oberflächenmerkmale (14) auch durch zumindest ein Durchgangsloch (16) geführt wird, um so eine verriegelte geschlossene Schleife zum Sichern von darin gehaltenem Körpergewebe zu bilden,
wobei die knotenlose Wundverschlussvorrichtung (10) eine bestimmte Geometrie aufweist, so dass jedes der Mehrzahl von Oberflächenmerkmalen (14) ausgestaltet ist zum in Verbindung bringen mit irgendeinem der Durchgangslöcher, wobei die Durchgangslöcher eindeutig passende Durchgangslöcher sind, die entlang der Länge des länglichen flexiblen Körpers (20) gebildet sind, so dass sie als ein unidirektionaler Verschlussmechanismus wirken.

2. Knotenlose Wundverschlussvorrichtung (10) nach Anspruch 1, wobei die Vorrichtung aus Materialien hergestellt ist, welche ausgewählt sind aus der Gruppe, die chirurgischer Fasern, chirurgisches Nahtmaterial, Fäden, Klebstreifen, Spaltbänder und Bänder umfasst.

3. Knotenlose Wundverschlussvorrichtung (10) nach Anspruch 1, wobei die Querschnittsgeometrie der Mehrzahl von Durchgangslöchern (16) ausgewählt ist aus der Gruppe, welche eine Schlüsselform, einen Keil, einen Kreis und einen Verbundkeil umfasst.

4. Knotenlose Wundverschlussvorrichtung (10) nach Anspruch 1, wobei die Querschnittsgeometrie des länglichen flexiblen Körpers (20) ausgewählt ist aus der Gruppe, welche ein Oval, ein Rechteck, eine Ellipse, einen Kreis, ein Quadrat, einen Stern, ein Trapez und ein Achteck umfasst.

5. Knotenlose Wundverschlussvorrichtung (10) nach Anspruch 1, wobei die Oberflächenmerkmale (14) ausgewählt sind aus einer Gruppe, welche Stachel, Haken, Schnapper, Vorsprünge, Blätter, Zähne und/oder Kombinationen davon, umfasst.

6. Knotenlose Wundverschlussvorrichtung (10) nach Anspruch 1, wobei das proximale Ende (12) eine daran befestigte Nadel (22) aufweist.

7. Knotenlose Wundverschlussvorrichtung (10) nach Anspruch 1, wobei die Oberflächenmerkmale (14) einen bioaktiven Wirkstoff innerhalb dem von dem Oberflächenmerkmal (14) und dem länglichen flexiblen Körper (20) eingeschlossenen Winkel aufweist.

8. Knotenlose Wundverschlussvorrichtung (10) nach Anspruch 7, wobei der bioaktive Wirkstoff ausgewählt ist aus der Gruppe, welche Antibiotika, Antimikrobiotika, Medikamente, Wachstumsbeschleuniger, Antigerinnungswirkstoffe, Anästhetika, Antiphlogistika, Wundheilwirkstoffe und Kombinationen daraus, umfasst.

## Revendications

1. Dispositif de fermeture de plaie sans noeud (10) comprenant :
un corps flexible allongé (20) ayant une extrémité proximale (12) et une extrémité distale (18), le corps flexible allongé (20) définissant un axe longitudinal (A-A); 1
une pluralité de caractéristiques de surface (14) s'étendant généralement à distance de l'axe longitudinal ;
une pluralité de trous de passage (16) formés le long de la longueur du corps flexible allongé (20) ;
dans lequel l'extrémité proximale (12) est configurée et dimensionnée pour passer à travers le tissu du corps et passer ensuite sélectivement à travers au moins l'un de la pluralité des trous de passage (16) de sorte qu'au moins l'une des caractéristiques de surface (14) passe également à travers le au moins un trou de passage (16) formant ainsi une boucle fermée bloquée pour fixer le tissu corporel maintenu à l'intérieur de cette dernière,
dans lequel le dispositif de fermeture de plaie sans noeud (10) a une géométrie spécifique de sorte que chacune de la pluralité des caractéristiques de surface (14) est adaptée pour s'interfacer avec l'un quelconque desdits trous de passage, dans lequel lesdits trous de passage correspondent uniquement aux trous de passage créés à travers la longueur du corps flexible allongé (20), afin de fonctionner comme un mécanisme de blocage unidirectionnel.

2. Dispositif de fermeture de plaie sans noeud (10) selon la revendication 1, dans lequel le dispositif est construit à partir de matériaux sélectionnés dans le groupe comprenant les fibres chirurgicales, les sutures, les filaments, les bandes, les feuilles fendues et les rubans.

3. Dispositif de fermeture de plaie sans noeud (10) selon la revendication 1, dans lequel la géométrie transversale de la pluralité de trous de passage (16) est choisie dans le groupe comprenant une forme de clé, un coin, un cercle et un coin multiple.

4. Dispositif de fermeture de plaie sans noeud (10) selon la revendication 1, dans lequel la géométrie transversale du corps flexible allongé (20) est choisie dans le groupe comprenant un ovale, un rectangle, une ellipse, un cercle, un carré, une étoile, une forme oblique et un octogone.

5. Dispositif de fermeture de plaie sans noeud (10) selon la revendication 1, dans lequel les caractéristiques de surface (14) sont choisies dans le groupe comprenant les ardillons, les crochets, les verrous, les saillies, les lamelles, les dents et/ou leurs combinaisons.

6. Dispositif de fermeture de plaie sans noeud (10) selon la revendication 1, dans lequel l'extrémité proximale (12) comprend en outre une aiguille (22) fixée à cette dernière.

7. Dispositif de fermeture de plaie sans noeud (10) selon la revendication 1, dans lequel les caractéristiques de surface (14) comprennent un agent bioactif dans un angle inclus de la caractéristique de surface (14) et le corps flexible allongé (20).

8. Dispositif de fermeture de plaie sans noeud (10) selon la revendication 7, dans lequel l'agent bioactif est choisi dans le groupe comprenant des biocides, des antibiotiques, des agents antimicrobiens, des médicaments, des facteurs de croissance, des agents anticoagulants, des anesthésiants, des agents anti-inflammatoires, des agents de réparation de plaie et leurs combinaisons.
